## Europäisches Patentamt
## European Patent Office
## Office européen des brevets

(11) Veröffentlichungsnummer : **0 075 145**
**B1**

(12)

# EUROPÄISCHE PATENTSCHRIFT

(45) Veröffentlichungstag der Patentschrift :
**17.10.84**

(51) Int. Cl.³ : **C 07 C 68/02, C 07 C 69/96**

(21) Anmeldenummer : **82107999.3**

(22) Anmeldetag : **31.08.82**

(54) **Verfahren zur kontinuierlichen Herstellung von Chlorameisensäurealkylestern.**

(30) Priorität : **10.09.81 DE 3135947**

(43) Veröffentlichungstag der Anmeldung :
**30.03.83 Patentblatt 83/13**

(45) Bekanntmachung des Hinweises auf die Patenterteilung : **17.10.84 Patentblatt 84/42**

(84) Benannte Vertragsstaaten :
**DE FR GB IT**

(56) Entgegenhaltungen :
**DE-A- 2 251 206**
**US-A- 2 778 846**

(73) Patentinhaber : **BAYER AG**
**Konzernverwaltung RP Patentabteilung**
**D-5090 Leverkusen 1 Bayerwerk (DE)**

(72) Erfinder : **Bauer, Dieter, Dr.**
**Bodelschwinghstrasse 127**
**D-4150 Krefeld (DE)**
Erfinder : **Dohm, Heinz, Dr.**
**Heyenbaumstrasse 86 b**
**D-4150 Krefeld (DE)**
Erfinder : **Schulte-Huermann, Werner, Dr.**
**Helmut-Macke-Strasse 10**
**D-4150 Krefeld (DE)**
Erfinder : **Hemmerich, Heinz-Peter, Dr.**
**Sebastian-Kneip-Weg 11**
**D-4150 Krefeld (DE)**

## Beschreibung

Die Erfindung betrifft ein Verfahren zur kontinuierlichen Herstellung von Chlorameisensäurealkylestern durch Umsetzung der entsprechenden Alkohole mit Phosgen.

Die Herstellung von aliphatischen Chlorameisensäureestern durch Umsetzung von Alkoholen mit Phosgen ist seit langem bekannt und wird industriell in großem Maßstab durchgeführt (s. z. B. Ullmanns Enzyklopädie der technischen Chemie, 4. Auflage, Band 9, Seite 381 ff (1975)).

So wird z. B. in der DE-PS 857 806 ein Verfahren zur Umsetzung von Säurehalogeniden, wie Phosgen, mit Hydroxylgruppen enthaltenden Verbindungen, wie Methanol und Ethanol, beschrieben, bei dem in Gegenwart von großoberflächigen Stoffen und bei Temperaturen, die vorzugsweise zwischen − 20 und − 70 °C liegen, gearbeitet wird (vgl. Ansprüche 1 und 2). Für dieses Verfahren ist es wesentlich, daß die Umsetzung bei möglichst tiefen Temperaturen und in Gegenwart von großoberflächigen Stoffen, wie Aktivkohle, durchgeführt wird, da sonst, beispielsweise bei 0 °C, erhebliche Mengen an Alkylchloriden und Dialkylcarbonaten gebildet werden und die Umsetzung nur unvollständig verläuft (s. Seite 3, Zeilen 60 bis 72 der DE-PS).

Weiterhin ist bekannt, Chlorameisensäureester durch Umsetzung von Phosgen mit Alkoholen herzustellen, wobei die Reaktion im Gegenstromverfahren durchgeführt wird. So wird in der US-PS 2 778 846 ein Verfahren beschrieben, bei dem man im Gegenstrom flüssigen Alkohol mit gasförmigem Phosgen in der Weise umsetzt, daß eine Temperaturdifferenz zwischen dem oberen Teil und dem unteren Teil der Gegenstromkolonne von 30 bis 50 °C besteht und in der Reaktionszone ein Überschuß an Alkohol vorhanden ist (vgl. Ansprüche 1 bis 6 der US-PS). In einer bevorzugten Ausführungsvariante des Verfahrens werden die Phosgen-haltigen Abgase, die bei der üblichen Umsetzung von Alkohol mit Phosgen in flüssiger Phase entstehen, in einer nachgeschalteten Kolonne im Gegenstrom mit überschüssigem Alkohol weiter umgesetzt (vgl. Ansprüche 9 bis 12 der US-PS).

Ein ähnliches Verfahren ist auch aus der FR-PS 1 336 606 bekannt. Dort wird zur Herstellung von Dialkylcarbonaten zunächst Alkohol mit Phosgen im Gegenstrom umgesetzt, wobei mindestens 1 Mol Alkohol pro Mol Phosgen eingesetzt wird. Gemäß dem Ausführungsbeispiel auf Seite 2 der französischen Patentschrift wird mit einem 2,5-fachen molaren Alkoholüberschuß gearbeitet.

Beide Verfahren haben jedoch beträchtliche Nachteile, die zum einen in der durch den sich in der Apparatur ausbildenden Temperaturgradienten erschwerten Steuerung und Kontrolle des Verfahrens liegen, zum anderen durch die Verringerung der Ausbeute und die Verminderung der Produktqualität bedingt sind.

Um dieses Nachteile bei der Herstellung von Chlorameinsensäureestern im Gegenstromverfahren zu vermeiden, wird gemäß der DE-AS 2 159 967 die kontinuierliche Herstellung von Chlorameisensäureestern dadurch bewerkstelligt, daß man die Reaktionsteilnehmer unter Ausnutzung des Dampfdruckes des Phosgens in die Aerosolphase überführt, die Umsetzung in der Aerosolphase unter isothermen Bedingungen im Gleichstrom durchführt und den Chlorameisensäureester von Chlorwasserstoff und überschüssigem Phosgen durch Aufheben des Aerosolzustandes und/oder Kondensation abtrennt (vgl. Anspruch 1 und Spalte 2, Zeilen 44 bis 68 sowie Spalte 3, Zeilen 1 bis 36 der DE-AS).

Nachteilig bei diesem Verfahren ist der Umstand, daß nur Produkte unzureichender Qualität erhalten werden. Der die Apparatur verlassende Chlorameisensäureester enthält nämlich noch Reste nicht umgesetzten Alkohols, der sich im bevorzugten Temperaturbereich von 35 bis 45 °C sofort weiter zu Dimethyl- bzw. Diethylcarbonat umsetzt. Ein weiterer Nachteil besteht darin, daß sich die Produktqualität infolge Hydrolyse deutlich verschlechterte, wenn, wie vorgeschlagen, ein Waschprozeß dem Verfahren nachgeschaltet würde (vgl. Spalte 5, Zeilen 55 bis 57).

Eine weitere Möglichkeit die Nachteile des Gegenstromverfahrens bei der Chlorameisensäureester-Herstellung zu vermeiden, wird auch in der DE-OS 2 704 262 beschrieben. Danach wird der Chlorameisensäuremethylester durch Umsetzung von Phosgen mit Methanol so hergestellt, daß man die Umsetzung in flüssiger Phase in Anwesenheit einer großen zirkulierenden Menge von vorgebildetem Chlorameisensäuremethylester ohne katalytische Materialien bei einer Temperatur, die konstant unter 20 °C liegt, durchführt; das Phosgen in dem vorgeformten Chlorameisensäuremethylester absorbiert und das Methanol rasch in einem fließenden Strom des vorgebildeten Chlorameisensäuremethylesters dispergiert, wobei die zirkulierende Menge sowie Wärmeaustauscher einen unerwünschten Temperaturanstieg verhindern (s. Anspruch 1 und Seite 4, mittlerer Absatz).

Dieses Verfahren hat den Nachteil, daß große Mengen Chlorameisensäuremethylester umgepumpt werden müssen, was zum einen enorme Pumpleistungen und entsprechend ausgelegte Apparaturen erfordert und daher die Wirtschaftlichkeit des Verfahrens beeinträchtigt und zum anderen sicherheitstechnische Probleme bezüglich der großen, vorhandenen Mengen an phosgenhaltigem Chlorameisensäuremethylester aufwirft.

Eine weitere Möglichkeit, Chlorkohlensäureester herzustellen ist u. a. in der DE-OS 2 251 206 und der DE-OS 2 453 284 beschrieben. Danach werden Phosgen und Alkohol einem Reaktionsgefäß, das im zeitlichen Gleichgewicht wenigstens 20 Gew.-% bzw. wenigstens 2 Gew.-% Phosgen im Reaktionsgemisch enthält, derart zugeführt, daß die Zeit für die homogene Einmischung des Alkohols in das Reaktionsgemisch weniger als 5, bzw. 1 Sekunde beträgt (vgl. die Ansprüche der o. g. deutschen Offenlegungsschriften).

Auch bei diesen Verfahren werden große Mengen an Chlorkohlensäureester umgewälzt, was, wie oben beschrieben, die Wirtschaftlichkeit beeinträchtigt und sicherheitstechnische Probleme aufwirft.

Es wurde nun ein Verfahren zur kontinuierlichen Herstellung von Chlorameisensäurealkylestern durch Umsetzung entsprechender Alkohole mit Phosgen im geringen Überschuß gefunden, das dadurch gekennzeichnet ist, daß man die Umsetzung im Gegenstrom bei konstanten Temperaturen im Bereich von 0 bis 30 °C so durchführt, daß im Reaktionsgemisch stets ein Phosgengehalt von 3 bis 20 Gew.-% vorliegt, daß das Reaktionsgemisch in einer nachgeschalteten Entgasungsstufe entgast wird und die dabei ausgetriebenen Gase wieder in die Reaktion zurückgeführt werden und daß aus dem bei der Umsetzung gebildeten Abgas nach Kühlung der Chlorameisensäureester sowie Phosgen abgeschieden und ebenfalls wieder in die Reaktion zurückgeführt werden.

Nach dem erfindungsgemäßen Verfahren werden bevorzugt Chlorameisensäuremethylester und Chlorameisensäureethylester hergestellt. Dazu wird Phosgen mit Methanol oder Ethanol umgesetzt. Nach dem erfindungsgemäßen Verfahren können jedoch auch andere Alkohole, wie Isopropanol, 2-Chlorethanol und 2-Ethoxyethanol zu den entsprechenden Chlorameisensäureestern umgesetzt werden.

Für die erfindungsgemäße, im Gegenstrom durchgeführte Umsetzung bei der der Alkohol in flüssiger Form in den oberen Teil der Reaktionsapparatur und das Phosgen dampfförmig in den unteren Teil der Reaktionsapparatur geleitet wird, ist es wesentlich, daß die Temperatur im Reaktor, d. h. in der Reaktionszone, während der Umsetzung konstant gehalten wird, was beispielsweise durch entsprechende Sole-Kühlung bewerkstelligt werden kann.

Falls die erfindungsgemäße Umsetzung mit Methanol durchgeführt wird, wird bevorzugt bei konstanten Temperaturen von 0 bis 15 °C, besonders bevorzugt bei 3 bis 10 °C, gearbeitet. Wird dagegen Phosgen mit Ethanol umgesetzt, arbeitet man bevorzugt bei 10 bis 30 °C, besonders bevorzugt bei 13 bis 20 °C.

Das erfindungsgemäße Verfahren wird zweckmäßigerweise in einem Druckbereich von etwa 0,8 bis 2 bar, bevorzugt 0,95 bis 1 bar, durchgeführt.

Das erfindungsgemäße Verfahren kann in den üblichen Gegenstromanlagen, z. B. in einer Füllkörperkolonne, die beispielsweise mit Füllkörpern aus keramischem Material gefüllt ist, durchgeführt werden.

Dem sich im unteren Teil des Reaktors ansammelnden, den Chlorameisensäureester enthaltenden Reaktionsgemisch, das stets einen Gehalt von 3 bis 20 Gew.-%, bevorzugt 8 bis 12 Gew.-%

Phosgen aufweist, wird ständig eine solche Menge Roh-Chlorameisensäureester entnommen, wie er sich nachbildet. Dadurch wird eine gleichbleibende Menge Reaktionsgemisch im unteren Teil des Reaktors aufrechterhalten, die von der Dimensionierung der Gegenstromanlage abhängt und z. B. bei einer Füllkörperkolonne mit gängiger Dimensionierung etwa 30 bis 250 l, bevorzugt 50 bis 150 l, beträgt.

Der im unteren Teil des Reaktors entnommene Roh-Chlormeinsensäureester wird in einer nachgeschalteten Entgasungsstufe entgast und die dabei ausgetriebenen Gase, die überwiegend aus Phosgen und Chlorwasserstoff neben wenig Alkylchlorid bestehen, werden in den unteren Teil des Reaktors zurückgeleitet und so der Reaktion wieder zugeführt.

Die Entgasung des aus dem unteren Teil des Reaktors entnommenen Reaktionsgemisches kann in bevorzugter Weise thermisch bei Temperaturen von etwa 60 bis 95 °C durchgeführt werden, wobei beim Chlorameisensäuremethylester im Temperaturbereich von 60 bis 75 °C und beim Chlorameisensäureethylester bei 70 bis 95 °C die Entgasung vorgenommen wird.

Es ist jedoch auch möglich, die Entgasung des Chlorameisensäureester enthaltenden Reaktionsgemisches auf andere Weise, z. B. durch Durchleiten von Inertgasen, wie Stickstoff, durch das Reaktionsgemisch gegebenenfalls bei erhöhter Temperatur, beispielsweise bei etwa 30 bis 95 °C, bevorzugt bei 40 bis 60 °C, zu bewerkstelligen.

Wird die nachgeschaltete Entgasung des Reaktionsgemisches bei erhöhter Temperatur durchgeführt, so ist es erforderlich, die ausgetriebenen Gase vor der Einleitung in den Reaktor auf nahezu Reaktionstemperatur (etwa 15 bis 50 °C) zu kühlen.

Der z. B. der nachgeschalteten Entgasungsstufe entnommene Chlorameisensäuremethylester bzw. Chlorameisensäureethylester besitzt eine Reinheit von ≧ 99 % und kann in dieser Form ohne zusätzliche Destillation direkt weiterverarbeitet werden.

Das bei der Umsetzung von Alkohol mit Phosgen sich bildende Abgas, bestehend im wesentlichen aus Chlorwasserstoff, Alkylchlorid, Phosgen und Chlormeinsäureester, das dem oberen Teil des Reaktors entnommen wird, wird nach Kühlung auf etwa −20 bis +15 °C, bevorzugt −10 bis +10 °C, einer Abscheidungsanlage zugeleitet, wo Chlorameisensäureester und Phosgen abgeschieden werden. Sowohl das abgeschiedene Phosgen als auch der abgeschiedene Chlorameisensäureester werden wieder in die Reaktion zurückgeführt.

Das erfindungsgemäße Verfahren kann allgemein wie folgt durchgeführt werden (erläutert für die Herstellung von Chlorameisensäuremethylester (MCF)):

Oberhalb des Flüssigkeitssumpfes einer Gegenstromrieselkolonne wird gasförmiges Phosgen eingeleitet. An Sumpf und Phosgeneinleitstelle schließen sich nach oben hin abwechselnd

mit keramischen Sattelkörpern gefüllte Kolonnenschüsse sowie mit Kühlsole betriebene Schlangenkühler an. Oberhalb eines solchen Kühlers erfolgt die Aufgabe des flüssigen Methanols.

Durch Regelung der Kühlleistung wird über die gesamte Höhe der Rieselkolonne ein praktisch lineares Temperaturprofil von etwa 7 °C eingestellt. Die Feinregulierung der Mengenströme der beiden Reaktanden geschieht derart, daß im Flüssigkeitssumpf der Apparatur stets ein roher Chlorameisensäuremethylester (MCF) mit einem Gehalt von ca. 10 % Phosgen anfällt.

Unter Konstanthaltung der Sumpfmenge wird nun ständig Rohchlorameisensäuremethylester aus dem Sumpf abgezogen und zwecks Entgasung auf eine kleine Füllkörperkolonne aufgegeben, deren Flüssigkeitssumpf durch Beheizung zu schwachem Sieden gebracht wird. Das Abgas der Entgasungskolonne wird nach Kühlung auf ca. 25 °C in den unteren Teil der Reaktionskolonne, etwa auf Höhe der Phosgeneinspeisung, zurückgeführt. Unten am Sumpf der Entgasungskolonne wird Reinchlorameisenssäuremethylester kontinuierlich im Maße des Anfalls abgezogen und nach Kühlung auf etwa 30 °C dem Lagerkessel für Reinprodukt zugeführt.

Das Abgas aus der Gegenstromrieselkolonne wird zunächst durch mit Sole beschickte Kühler auf etwa 0 °C abgekühlt. Dabei kann der Abgaskühler sowohl als Rückflußkühler unmittelbar auf die Reaktionskolonne aufgesetzt sein als auch neben dieser aufgebaut sein, wenn die Rückführung des Flüssigkeitsanfalls in den Reaktionsteil gewährleistet ist. Das den Abgaskühler verlassende Abgas wird über einen geeigneten Flüssigkeitsabscheider, beispielsweise einen Zyklon, sowie einen Tröpfchenabscheider (Demister) weiter aufgearbeitet. Der Flüssigkeitsanfall beider Apparate wird in den unteren Teil der Reaktionskolonne, etwa auf Höhe der Phosgeneinspeisung, zurückgeführt. Schließlich gelangt das Abgas in eine geeignete Entsorgungsanlage.

Gegenüber den aus dem Stand der Technik bekannten Verfahren liefert das erfindungsgemäße Verfahren höhere Ausbeuten und eine verbesserte Produktqualität an Chlorameisensäurealkylester. So werden nach dem erfindungsgemäßen Verfahren z. B. Ausbeuten an Chlorameisensäuremethylester bzw. Chlorameisensäureethylester von ≧ 94 % der Theorie erzielt mit einer Reinheit von ≧ 99 %. Weiterhin wird bei dem erfindungsgemäßen Verfahren die Umwälzung großer Mengen an Chlorameisensäureester vermieden, wodurch sich das erfindungsgemäße Verfahren besonders wirtschaftlich gestaltet und sicherheitstechnisch einfacher zu handhaben ist.

Daß nach dem erfindungsgemäßen, im Gegenstrom durchgeführten Verfahren solch gute Ausbeuten und hohe Reinheiten an Chlorameisensäureestern erhalten werden, ist besonders im Hinblick auf die DE-AS 2 159 967 und die DE-OS 2 704 262 überraschend, da dort betont wird, daß beim Gegenstromverfahren nur verminderte Ausbeuten und Reinheiten an Chlorameisensäureester erzielt werden, die außerdem noch mit anderen, beträchtlichen Nachteilen verbunden sind.

Das erfindungsgemäße Verfahren sei anhand der folgenden Beispiele erläutert.

Beispiel 1

Chlorameisensäuremethylester (MCF)

In eine Gegenstromrieselkolonne wird oberhalb des Flüssigkeitssumpfes gasförmiges Phosgen (ca. 92 kg/h) eingeleitet. An Sumpf und Phosgeneinleitstelle schließen sich nach oben hin abwechselnd mit keramischen Sattelkörpern gefüllte Kolonnenschüsse sowie mit Kühlsole betriebene Schlangenkühler an. Oberhalb eines solchen Kühlers erfolgt die Aufgabe von ca. 26 kg/h flüssigem Methanol. Durch Regelung der Kühlleistung wird über die gesamte Höhe der Rieselkolonne ein praktisch lineares Temperaturprofil von ca. 7 °C eingestellt. Die Feinregulierung der Mengenströme der beiden Reaktanden geschieht derart, daß im Flüssigkeitssumpf der Apparatur stets ein roher Chlorameisensäuremethylester mit einem Gehalt von ca. 10 % Phosgen anfällt.

Unter Konstanthaltung der Sumpfmenge wird nun ständig Roh-Chlorameisensäuremethylester aus dem Sumpf abgezogen und zwecks Entgasung auf eine kleine Füllkörperkolonne aufgegeben, deren Flüssigkeitssumpf durch Beheizung zu schwachem Sieden gebracht wird. Das Abgas der Entgasungskolonne wird nach Kühlung auf ca. 25 °C in den unteren Teil der Reaktionskolonne, etwa auf Höhe der Phosgeneinspeisung, zurückgeführt. Unten am Sumpf der Entgasungskolonne wird Rein-Chlorameisensäuremethylester kontinuierlich in einer Menge von ca. 73 kg/h abgezogen, dessen Gehalt gaschromatographisch zu 99,1 % bestimmt wurde. Das Produkt war praktisch frei von Phosgen und Chlorwasserstoff.

Das Abgas aus der Gegenstromrieselkolonne wird zunächst durch mit Sole beschickte Kühler auf ca. 0 °C abgekühlt. Dabei kann der Abgaskühler sowohl als Rückflußkühler unmittelbar auf die Reaktionskolonne aufgesetzt sein als auch neben diese aufgebaut sein, wenn die Rückführung des Flüssigkeitsanfalls in den Reaktionsteil gewährleistet ist. Das den Abgaskühker verlassende Abgas wird über einen geeigneten Flüssigkeitsabscheider, beispielsweise einen Zyklon, sowie einen Tröpfchenabscheider weiter aufgearbeitet. Das Flüssigkeitsanfall beider Apparate wird in den unteren Teil der Reaktionskolonne, etwa auf Höhe der Phosgeneinspeisung, zurückgeführt. Das Abgas wird schließlich einer Entsorgungsanlage zugeführt.

Beispiel 2

Chlorameisensäureethylester (ECF)

Analog zu Beispiel 1 wurden jetzt ca. 80 kg/h gasförmiges Phosgen sowie ca. 31,5 kg/h flüssiges Ethanol in die Gegenstromanlage eingespeist. Über die gesamte Höhe der Reaktionskolonne wurde gleichbleibend eine Temperatur von etwa 17 °C aufrechterhalten. Das Abgas aus der Rieselkolonne wurde auf ca. 10 °C abgekühlt bevor es der Flüssigkeits- und Tröpfchenabscheidung zugeleitet wurde. Alle übrigen Bedingungen entsprachen denen von Beispiel 1. Am Boden der Entgasungskolonne wurden direkt ca. 70 kg/h Rein-ECF mit einem Gehalt von 99,5 % abgezogen. Das Produkt war praktisch frei von Phosgen und Chlorwasserstoff.

**Ansprüche**

1. Verfahren zur kontinuierlichen Herstellung von Chlorameisensäurealkylestern durch Umsetzung entsprechender Alkohole mit Phosgen in geringem Überschuß, dadurch gekennzeichnet, daß man die Umsetzung im Gegenstrom bei konstanten Temperaturen im Bereich von 0 bis 30 °C so durchführt, daß im Reaktionsgemisch stets ein Phosgengehalt von 3 bis 20 Gew.-% vorliegt, daß das Reaktionsgemisch in einer nachgeschalteten Entgasungsstufe entgast wird und die dabei ausgetriebenen Gase wieder in die Reaktion zurückgeführt werden und daß aus dem bei der Umsetzung gebildeten Abgas nach Kühlung der Chlorameisensäureester sowie Phosgen abgeschieden und ebenfalls wieder in die Reaktion zurückgeführt werden.

2. Verfahren nach Anspruch 1, dadurch gekennzeichnet, daß man die Umsetzung in einem Druckbereich von 0,8 bis 2 bar durchführt.

3. Verfahren nach Anspruch 1, dadurch gekennzeichnet, daß man die Umsetzung in einem Druckbereich von 0,95 bis 1 bar durchführt.

4. Verfahren nach Ansprüchen 1 bis 3, dadurch gekennzeichnet, daß man die Umsetzung so durchführt, daß im Reaktionsgemisch stets ein Phosgengehalt von 8 bis 12 Gew.-% vorliegt.

5. Verfahren nach Ansprüchen 1 bis 4, dadurch gekennzeichnet, daß das Reaktionsgemisch in einer nachgeschalteten Entgasungsstufe bei Temperaturen von 60 bis 95 °C entgast wird.

6. Verfahren nach Ansprüchen 1 bis 4, dadurch gekennzeichnet, daß das Reaktionsgemisch in einer nachgeschalteten Entgasungsstufe durch Durchleiten von Inertgasen bei Temperaturen von 30 bis 95 °C entgast wird.

7. Verfahren nach Ansprüchen 1 bis 6, dadurch gekennzeichnet, daß die bei der Entgasungsstufe ausgetriebenen Gase vor der Rückführung in die Reaktion auf Temperaturen von 15 bis 50 °C gekühlt werden.

**Claims**

1. A process for the continuous preparation of alkyl chloroformates by the reaction of appropriate alcohols with a small excess of phosgene, characterised in that the reaction is carried out by a countercurrent method at constant temperatures in the range of 0 to 30 °C in such a manner that a phosgene content of 3 to 20 % by weight is always present in the reaction mixture, the reaction mixture is degassed in a downstream degassing stage and the gases expelled in this procedure are recycled to the reaction, and the chloroformic acid ester and phosgene are separated from the exit gas formed in the reaction, after this gas has been cooled, and are likewise recycled to the reaction.

2. Process according to Claim 1, characterised in that the reaction is carried out in a pressure range from 0.8 to 2 bar.

3. Process according to Claim 1, characterised in that the reaction is carried out in a pressure range from 0.95 to 1 bar.

4. Process according to Claims 1 to 3, characterised in that the reaction is carried out in such a manner that a phosgene content of 8 to 12 % by weight is always present in the reaction mixture.

5. Process according to Claims 1 to 4, characterised in that the reaction mixture is degassed in a downstream degassing stage at temperatures of 60 to 95 °C.

6. Process according to Claims 1 to 4, characterised in that the reaction mixture is degassed in a downstream degassing stage by passing inert gases through the mixture at temperatures of 30 to 95 °C.

7. Process according to Claims 1 to 6, characterised in that the gases expelled in the degassing stage are cooled to temperatures of 15 to 50 °C before being recycled to the reaction.

**Revendications**

1. Procédé de production continue d'esters alkyliques d'acide chloroformique par réaction d'alcools correspondants avec le phosgène en léger excès, caractérisé en ce qu'on conduit la réaction à contre-courant à températures constantes dans l'intervalle de 0 à 30 °C de manière qu'il y ait toujours dans le mélange réactionnel une teneur en phosgène de 3 à 20 % en poids, en ce que le mélange réactionnel est dégazé dans un étage de dégazage monté à la suite et les gaz ainsi chassés sont recyclés dans la réaction et en ce que l'ester d'acide chloroformique est séparé de même que du phosgène du gaz résiduel formé dans la réaction, après refroidissement, et est également recyclé dans la réaction.

2. Procédé suivant la revendication 1, caractérisé en ce qu'on conduit la réaction dans un intervalle de pression de 0,8 à 2 bars.

3. Procédé suivant la revendication 1, caractérisé en ce qu'on conduit la réaction dans un intervalle de pression de 0,95 à 1 bar.

4. Procédé suivant les revendications 1 à 3, caractérisé en ce qu'on conduit la réaction de manière qu'il y ait toujours dans le mélange réactionnel une teneur en phosgène de 8 à 12 % en poids.